# EUROPÄISCHE PATENTSCHRIFT

(11) **EP 1 078 916 B1**
(45) Veröffentlichungstag und Bekanntmachung des Hinweises auf die Patenterteilung: **11.12.2002**
(21) Anmeldenummer: 00115618.1
(22) Anmeldetag: 20.07.2000
(51) Int. Cl.: C07C 231/08, C07C 233/18, C07C 233/47

(54) **Verfahren zur Herstellung von N-Acyl-aminosäureestern und N-Acyl-aminoacetalen**
Process for the preparation of N-acylaminoacid esters and N-acylaminoacetals
Procédé de préparation d'esters d'acides N-acylaminés et de N-acylaminoacétals

(30) Priorität: 26.08.1999 DE 19940641
(43) Veröffentlichungstag der Anmeldung: 28.02.2001
(73) Patentinhaber: BASF AKTIENGESELLSCHAFT, 67056 Ludwigshafen (DE)
(72) Erfinder: Paust, Joachim, Dr., 67141 Neuhofen (DE); Ernst, Hansgeorg, Dr., 67346 Speyer (DE); Kaczmarek, Reinhard, Dr., 67454 Hassloch (DE); Jaedicke, Hagen, Dr., 67069 Ludwigshafen (DE)

(56) Entgegenhaltungen:
- EP-A- 0 332 083
- EP-A- 0 709 367
- WO-A-96/24074
- DE-A- 4 233 771
- DE-B- 1 117 134
- GB-A- 2 252 770
- MICHAELIDES, M.R. ET AL.: "Synthesis and Pharmacological Evaluation of 1-(Aminomethyl)-3,4-dihydro-5-hydroxy-1H-2 -benzopyrans as Dopamine D1 Selective Ligands" JOURNAL OF MEDICINAL CHEMISTRY, Bd. 34, Nr. 10, 1991, Seiten 2946-2953, XP000941382
- PLAQUEVENT, J.-C.: "Création non Classique de Liaisons Peptidiques par Isomérisation Oxaziridine-Amide : Etudes Modèles et Synthèse Formelle de l'Aspartame" NEW JOURNAL OF CHEMISTRY, Bd. 15, Nr. 7, 1991, Seiten 579-585, XP000941383
- SHOJI, S. ET AL.: "N-Fatty Acyl Compounds Inhibit Myristoyl Acylation of pp60v-src and Reduce Tumorigenecity of Rous Sarcoma Virus-Infected Cells" BIOCHEMISTRY INTERNATIONAL, Bd. 23, Nr. 1, 1991, Seiten 15-23, XP000942938
- BUCK, K.: "Photochemie von Acylaziden. VIII. Reagieren Acylnitrene wie 1,3-Dipole ?" JOURNAL FÜR PRAKTISCHE CHEMIE / CHEMIKER-ZEITUNG, Bd. 336, Nr. 8, 1994, Seiten 678-685, XP000942961
- AIZUPURUA, J.M., PALOMO, C.: "Reagents and Synthetic Methods 30. Practical and Improved Method for Formylating Amino Compounds by means of Formic Acid-Dimethylformamide System" SYNTHETIC COMMUNICATIONS, Bd. 13, Nr. 9, 1983, Seiten 745-752, XP000943114

## Beschreibung

Die Erfindung betrifft ein Verfahren zur Herstellung von N-Acyl-aminosäureestern und N-Acyl-aminoacetalen.

Für die Synthese von Aminosäuren und deren Ester sind eine Vielzahl von unterschiedlichen Methoden bekannt. Eine Übersicht findet sich u.a. in Ullmanns Encyclopedia of Industrial Chemistry, Vol. A2, 57-97, VCH Weinheim 1985.

Technische Synthesen für D,L-α-Aminosäuren, beispielsweise die Strecker-Synthese, gehen von Aldehyden aus, die mit NH₃ und HCN zu Aminonitrilen umgesetzt werden. Die Nitrilgruppe läßt sich anschließend mit Alkoholen oder Wasser zu den entsprechenden Estern bzw. Aminosäuren umsetzen.

DE-A-3145736 beschreibt ein Verfahren zur Herstellung von N-Formyl-α-aminosäureestern durch Umsetzung von Aminonitrilen - z.B. aus der Strecker Synthese - mit einem entsprechenden Alkohol und Formamid in Gegenwart von Chlorwasserstoff.

Bekannt ist ferner die Herstellung von N-Formyl-D,L-alanin aus Brenztraubensäure durch Kochen mit Ammoniumformiat in Ameisensäure [F. Yoneda und K. Kuroda, J. Chem. Soc. Chem. Commun., 1982, 927-929].

N-Formylalaninester werden unter anderem zur Herstellung von Vitamin-B6 (Pyridoxin) [Übersicht von König und Böll, Chem. Ztg. 100, 107/8 (1976)] und Isocyansäure z.B. nach Ugi, Angew. Chem. 77, 492 (1965) verwendet.

Die beschriebenen Verfahren haben den Nachteil, daß die Ausgangsstoffe fertige Aminosäuren oder deren Vorstufen - beispielsweise Cyanhydrine oder Aminonitrile aus der Strecker-Synthese - sind, die zuvor in einem eigenen Verfahren hergestellt werden müssen.

GB-A-2252770 betrifft ein Verfahren zur Herstellung von N-Acylalpha-aminosäuren durch Reaktion von Kohlenmonoxid mit einem Aldehyd und einem Carbonsäureamid, wobei die Reaktion katalysiert wird durch ein Gemisch aus einer Verbindung, enthaltend ein Metall der Eisengruppe, und einer Säure, beispielsweise, Sulfonsäure, carbonsäure, Phosphorsäure oder Halogenwasserstoffsäure.

EP-A-0 709 367 offenbart ein Verfahren zur Herstellung von N-Alkenylcarbonsäureamiden durch Umsetzung eines Carbonsäureamids un einer Carbonylverbindung in Gegenwart einer Base.

EP-A-0 332 083 beinhaltet die Synthese sekundärer Formamide durc Umsetzung von Acetalen mit Formamid in Gegenwart einer starken Säure.

in WO 96/24074 wird die Verwendung von N-[2,2-Dialkoxyalkyl]acetamide zur Quervernetzung von Polyvinylalkoholen beschrieben.

DE-A-42 33 771 beschreibt ein zweistufiges Verfahren zur Herstel lung von 2-substituierten Oxazolen, wobei eine Carbonsäure oder ein Carbonsäureester mit einem Aminoacetal zu einem Amidoacetal kondensiert wird und das Amidoacetal anschließend in Polyphosphorsäure zum Oxazol cyclisiert.

Michaelides et al., J. Med. Chem. (1991), 34 (10), 2946-2953 beschreibt die Verwendung von N-Formylaminoacetaldehyddimethylacetal zur Herstellung von Benzopyranderivaten.

Plaquevent et al., New J. Chem. (1991), 15, 579-585 beschreibt die Bildung von N-Acylaminoacetaldehyddimethylacetalen durch Iso merisierung der entsprechenden Oxaziridine.

Buck et al., J. Prakt. Chem. (1994), 336(8), 678-685, beschreibt die photochemische Synthese von N-(2-Ethoxy-2-methoxyethyl)-benzamid und N-(2-Butoxy-2-methoxyethyl)-benzamid.

Aufgabe der Erfindung war es, ein technisch einfach durchführbares Verfahren zur Herstellung von N-Acyl-aminosäureestern und N-Acyl-aminoacetalen unter Verwendung gut zugänglicher Ausgangsstoffe bereitzustellen.

Diese Aufgabe wurde gelöst durch ein Verfahren zur Herstellung von N-Acyl-Derivaten der allgemeinen Formel I, 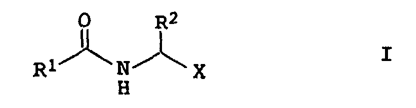 in der die Substituenten unabhängig voneinander folgende Bedeutung haben:
- X: CH(OR³)₂, COOR³;
- R¹: Wasserstoff, C₁-C₁₂-Alkyl, Aryl, gegebenenfalls substituiert;
- R²: Wasserstoff, C₁-C₁₂-Alkyl, Aryl, gegebenenfalls substituiert;
- R³: C₁-C₁₂-Alkyl,
dadurch gekennzeichnet, daß man ein Carbonsäureamid R¹-CONH₂ der allgemeinen Formel II mit einem Glyoxalmonoacetal-Derivat der allgemeinen Formel III, 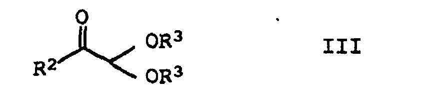 in Gegenwart einer Carbonsäure R⁴-COOH der allgemeinen Formel IV mit R⁴ = Wasserstoff oder C₁-C₁₂-Alkyl umsetzt, wobei die Substituenten R¹ bis R³ die bereits oben genannte Bedeutung haben.

Als Alkylreste für R¹ bis R⁴ seien verzweigte oder unverzweigte C₁-C₁₂-Alkylketten, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl, n-Undecyl und n-Dodecyl genannt.

Die oben genannten Alkylketten können gegebenenfalls hydroxyliert oder mit Mercaptogruppen substituiert sein. Als Beispiele seien bevorzugt Hydroxymethyl-, Hydroxyethyl- wie [CH₃-CH(OH)- bzw. CH₂(OH)-CH₂-] oder Mercaptomethylreste genannt.

Für den Fall, daß der Rest X in der Formel I die Bedeutung CH(OR³)₂ hat, können die Substituenten R³ zusammen mit den Sauerstoffatomen, an denen sie gebunden sind, auch einen 5- oder 6-Ring bilden. Als Ausgangsverbindung werden hierbei beispielsweise cyclische Glyoxalmonoacetale der allgemeinen Formel IIIa bis IIIc eingesetzt.

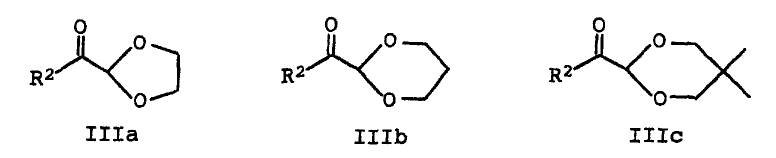

Unter Aryl für R¹ und R² sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können.

Als bevorzugte Reste für R¹ seien Wasserstoff sowie die in der o.g. Liste erwähnten verzweigten oder unverzweigten C₁-C₈-Alkylketten, besonders bevorzugt C₁-C₃-Alkylketten genannt. Ganz besonders bevorzugte Reste für R¹ sind Wasserstoff, Methyl und Ethyl.

Als bevorzugte Reste für R² seien Phenyl sowie aus der o.g. Liste die verzweigten oder unverzweigten C₁-C₈-Alkylketten, besonders bevorzugt C₁-C₃-Alkylketten genannt. Ein ganz besonders bevorzugter Rest für R² ist Methyl.

Als bevorzugte Alkylreste für R³ seien aus der o.g. Liste die verzweigten oder unverzweigten C₁-C₈-Alkylketten, besonders bevorzugt C₃-C₈-Alkylketten wie z.B. n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, n-Heptyl, n-Octyl oder 2-Ethylhexyl genannt.

Als bevorzugte Reste für R⁴ seien aus der o.g. Liste die verzweigten oder unverzweigten C₁-C₈-Alkylketten, besonders bevorzugt C₁-C₃-Alkylketten genannt. Ganz besonders bevorzugte Reste für 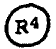 sind Methyl, Ethyl, n-Propyl und iso-Propyl.

Je nach Menge an eingesetztem Carbonsäureamid R¹-CONH₂ sowie an eingesetzter Carbonsäure R⁴-COOH läßt sich die Bildung der unterschiedlichen N-Acyl-Derivate der Formel I gezielt steuern.

So wurde überraschend gefunden, daß die Reaktion bei einer Menge an eingesetztem Carbonsäureamid R¹-CONH₂ und eingesetzter Carbonsäure R⁴-COOH von jeweils 250 bis 800 Mol-%, bevorzugt 400 bis 600 Mol-%, bezogen auf das eingesetzte Acetal der allgemeinen Formel II, N-Acyl-aminosäureestern der Formel I mit X = COOR³ liefert.

Als besonders vorteilhafte Ausführungsform des Verfahrens hat sich der Einsatz des Carbonsäureamids R¹-CONH₂ und der Carbonsäure R⁴-COOH zu gleichen molaren Anteilen erwiesen.

Das erfindungsgemäße Verfahren eignet sich hier insbesondere zur Herstellung von N-Formyl-α-amino-propionsäureester der allgemeinen Formel Ia, 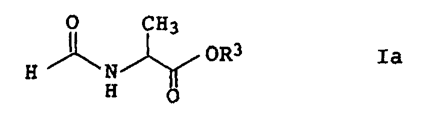 in der der Substituent R³ die Bedeutung C₁-C₈-Alkyl, bevorzugt C₃-C₈-Alkyl hat.

Die Bildung der N-Acyl-aminoacetale der allgemeinen Formel I mit X = CH(OR³)₂ ist bevorzugt, wenn die Menge an eingesetztem Carbonsäureamid R¹-CONH₂ und eingesetzter Carbonsäure R⁴-COOH jeweils 50 bis 250 Mol-%, bevorzugt 100 bis 200 Mol-%, bezogen auf das eingesetzte Acetal der allgemeinen Formel II, beträgt. Auch in diesem Fall ist es besonders vorteilhaft, wenn das Carbonsäureamid R¹-CONH₂ und die Carbonsäure R⁴-COOH im Molverhältnis 1:1 in die Reaktion eingesetzt werden.

Im Falle der N-Acyl-aminoacetale der allgemeinen Formel I eignet sich das erfindungsgemäße Verfahren vorteilhafterweise zur Herstellung der N-Formyl-2-amino-propionaldehydacetale der allgemeinen Formel Ib, 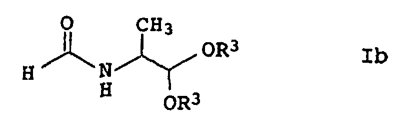 in der der Substituent R³ die Bedeutung C₁-C₈-Alkyl, bevorzugt C₃-C₈-Alkyl hat.

Die Umsetzung sowohl zu den N-Acyl-aminosäureestern als auch zu den N-Acyl-aminoacetalen wird bei einer Temperatur von 40 bis zu 200°C, bevorzugt 60 bis 150°C durchgeführt.

Die Reaktion erfolgt erfindungsgemäß im Druckbereich zwischen 200 und 1000 mbar und wird bevorzugt zwischen 500 und 1000 mbar, besonders bevorzugt bei Normaldruck ausgeführt.

Die Reaktion kann mit und ohne zusätzlichem Lösungsmittel gefahren werden. Bevorzugt erfolgt die Reaktion ohne Zusatz eines Lösungsmittels.

Das erfindungsgemäße Verfahren läßt sich außerdem vorteilhafterweise als sog. "Eintopfverfahren" durchführen und liefert die N-Acyl-aminosäureestern als auch die neuen N-Acyl-aminoacetalen in ausgezeichneten Ausbeuten.

Die Isolierung des gewünschten Endprodukts erfolgt in an sich bekannter Weise. Im Falle von flüssigen Reaktionsprodukten erfolgt in der Regel eine destillative Aufreinigung der gebildeten Ester bzw. Acetale.

Anhand der folgenden Beispiele soll der Gegenstand der vorliegenden Erfindung näher erläutert werden.

### Beispiel 1

### N-Formyl-D,L-alaninbutylester aus Methylglyoxal-di-n-butylacetal

100 g Methylglyoxaldibutylacetal (Reinheit 93,5 %, hergestellt nach EP 036539) wurden mit 100 g Formamid gemischt und innerhalb von 10 min. mit 100 g Ameisensäure versetzt. Das Gemisch erwärmte sich auf 40°C und wurde dann in 20 min auf Rückflußtemperatur erhitzt. Nach 2 Stunden Reaktionszeit wurde die auf Raumtemperatur abgekühlte Reaktionsmischung mit verdünnter Sodalösung gewaschen und das gewünschte Produkt im Vakuum bei 2 mbar destilliert. Man erhielt 74,5 g reinen N-Formyl-D,L-alaninbutylester (93% d.T.).

### Beispiel 2

### N-Formyl-D,L-alanin-2-ethylhexylester aus Methylglyoxaldi-2-ethylhexylacetal

50 g Methylglyoxal-di-2-ethylhexylacetal (Reinheit 92%) wurden mit 30 g Formamid und 30 g Ameisensäure 2,5 Stunden unter Rückfluß gekocht. Man wusch mit 200 ml Sodalösung und destillierte. In der Hauptfraktion isolierte man 29,8 g N-Formy-D,L-lalanin-2-ethyl-hexylester (89% d.T.).

### Beispiel 3

### N-Formylamino-propionaldehyd-di-n-butylacetal aus Methylglyoxal-di-n-butylacetal

100 g Methylglyoxaldibutylacetal (Reinheit 93,5 %, hergestellt nach EP 036539) wurden mit 50 g Formamid gemischt und innerhalb von 10 min. mit 50 g Ameisensäure versetzt. Das Gemisch erwärmte sich auf 40°C und wurde dann in 20 min auf Rückflußtemperatur erhitzt. Nach 2 Stunden Reaktionszeit wurde die auf Raumtemperatur abgekühlte Reaktionsmischung mit verdünnter Sodalösung gewaschen und das gewünschte Produkt im Vakuum bei 2 mbar destilliert. Man erhielt 39 g N-Formylamino-propionaldehyd-di-n-butylacetal.

## Patentansprüche

1. Verfahren zur Herstellung von N-Acyl-Derivaten der allgemeinen Formel I, 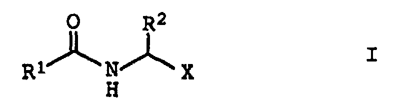 in der die Substituenten unabhängig voneinander folgende Bedeutung haben:
X CH(OR³)₂, COOR³;
R¹ Wasserstoff, C₁-C₁₂-Alkyl, Aryl, gegebenenfalls substituiert;
R² Wasserstoff, C₁-C₁₂-Alkyl, Aryl, gegebenenfalls substituiert;
R³ C₁-C₁₂-Alkyl,
wobei für den Fall, daß der Rest X die Bedeutung CH(OR³)₂ hat, können die Substituenten R³ zusammen mit den Sauerstoffatomen, an denen sie gebunden sind, auch einen 5-oder 6-Ring bilden,
**dadurch gekennzeichnet, daß** man ein Carbonsäureamid R¹-CONH₂ der allgemeinen Formel II mit einem Glyoxalmonoacetal-Derivat der allgemeinen Formel III, 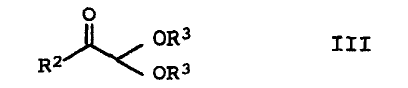 in Gegenwart einer Carbonsäure R⁴-COOH der allgemeinen Formel IV mit R⁴ = Wasserstoff oder C₁-C₁₂-Alkyl umsetzt, wobei die Substituenten R¹ bis R³ die bereits oben genannte Bedeutung haben.

2. Verfahren nach Anspruch 1, **dadurch gekennzeichnet, daß** die Substituenten folgende Bedeutung haben:
R¹ Wasserstoff, C₁-C₈-Alkyl;
R² C₁-C₈-Alkyl, Aryl, gegebenenfalls substituiert;
R³ und R⁴ C₁-C₈-Alkyl.

3. Verfahren nach Anspruch 2, **dadurch gekennzeichnet, daß** die Substituenten folgende Bedeutung haben:
R¹ Wasserstoff;
R² bis R⁴ C₁-C₈-Alkyl.

4. Verfahren nach einem der Ansprüche 2 oder 3 zur Herstellung von N-Formyl-α-amino-propionsäureester der allgemeinen Formel Ia, 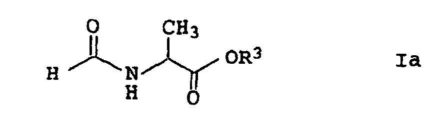 in der der Substituent R³ die Bedeutung C₁-C₈-Alkyl hat.

5. Verfahren nach Anspruch 1, **dadurch gekennzeichnet, daß** die Substituenten folgende Bedeutung haben:
X CH(OR³)₂;
R¹ Wasserstoff, C₁-C₈-Alkyl;
R² C₁-C₈-Alkyl, Aryl, gegebenenfalls substituiert;
R³ und R⁴ C₁-C₈-Alkyl.

6. Verfahren nach Anspruch 5, **dadurch gekennzeichnet, daß** die Substituenten folgende Bedeutung haben:
R¹ Wasserstoff;
R² bis R⁴ C₁-C₈-Alkyl.

7. Verfahren nach einem der Ansprüche 5 oder 6 zur Herstellung von N-Formyl-2-amino-propionaldehydderivaten der allgemeinen Formel Ib, 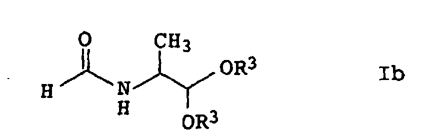 in der der Substituent R³ die Bedeutung C₁-C₈-Alkyl hat.

8. Verfahren nach einem der Ansprüche 2 bis 4, **dadurch gekennzeichnet, daß** die Menge an jeweils eingesetztem Carbonsäureamid R¹-CONH₂ und eingesetzter Carbonsäure R⁴-COOH 250 bis 800 Mol-%, bezogen auf das eingesetzte Acetal der allgemeinen Formel II, beträgt.

9. Verfahren nach Anspruch 8, **dadurch gekennzeichnet, daß** die Menge an jeweils eingesetztem Carbonsäureamid R¹-CONH₂ und eingesetzter Carbonsäure R⁴-COOH 400 bis 600 Mol-%, bezogen auf das eingesetzte Acetal der allgemeinen Formel II, beträgt.

10. Verfahren nach einem der Ansprüche 8 oder 9, **dadurch gekennzeichnet, daß** das Carbonsäureamid R¹-CONH₂ und die Carbonsäure R⁴-COOH im Molverhältnis 1:1 in der Reaktion eingesetzt werden.

11. Verfahren nach einem der Ansprüche 5 bis 7, **dadurch gekennzeichnet, daß** die Menge an jeweils eingesetztem Carbonsäureamid R¹-CONH₂ und eingesetzter Carbonsäure R⁴-COOH 50 bis 250 Mol-%, bezogen auf das eingesetzte Acetal der allgemeinen Formel II, beträgt.

12. Verfahren nach Anspruch 11, **dadurch gekennzeichnet, daß** die Menge an jeweils eingesetztem Carbonsäureamid R¹-CONH₂ und eingesetzter Carbonsäure R⁴-COOH 100 bis 200 Mol-%, bezogen auf das eingesetzte Acetal der allgemeinen Formel II, beträgt.

13. Verfahren nach einem der Ansprüche 11 oder 12, **dadurch gekennzeichnet, daß** das Carbonsäureamid R¹-CONH₂ und die Carbonsäure R⁴-COOH im Molverhältnis 1:1 in der Reaktion eingesetzt werden.

## Claims

1. A process for preparing N-acyl derivatives of the formula I, 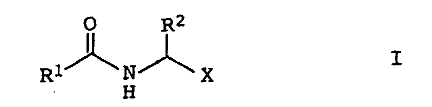 in which the substituents independently of one another have the following meanings:
X is CH(OR³)₂, COOR³;
R¹ is hydrogen, C₁-C₁₂-alkyl, aryl, unsubstituted or substituted;
R² is hydrogen, C₁-C₁₂-alkyl, aryl, unsubstituted or substituted;
R³ is C₁-C₁₂-alkyl,
wherein, if the radical X is CH(OR³)₂, the substituents R³ together with the oxygen atoms to which they are bonded may also form a 5- or 6-membered ring,
which comprises reacting a carboxamide R¹-CONH₂ of the formula II with a glyoxal monoacetal derivative of the formula III, 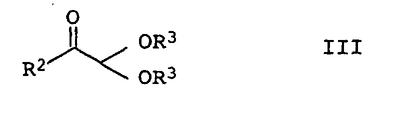 in the presence of a carboxylic acid R⁴-COOH of the formula IV where R⁴ = hydrogen or C₁-C₁₂-alkyl, where the substituents R¹ to R³ are as defined above.

2. A process as claimed in claim 1, wherein the substituents have the following meanings:
R¹ is hydrogen, C₁-C₈-alkyl;
R² is C₁-C₈-alkyl, aryl, unsubstituted or substituted;
R³ and R⁴ are C₁-C₈-alkyl.

3. A process as claimed in claim 2, wherein the substituents have the following meaning:
R¹ is hydrogen;
R² to R⁴ are C₁-C₈-alkyl.

4. A process as claimed in any of claims 2 or 3 for preparing N-formyl-α-aminopropionic acid esters of the formula Ia 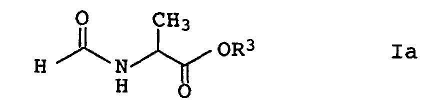 in which the substituent R³ is C₁-C₈-alkyl.

5. A process as claimed in claim 1, wherein the substituents have the following meanings:
X is CH(OR³)₂;
R¹ is hydrogen, C₁-C₈-alkyl;
R² is C₁-C₈-alkyl, aryl, unsubstituted or substituted;
R³ and R⁴ are C₁-C₈-alkyl.

6. A process as claimed in claim 5, wherein the substituents have the following meanings:
R¹ is hydrogen;
R² to R⁴ are C₁-C₈-alkyl.

7. A process as claimed in either of claims 5 or 6 for preparing N-formyl-2-aminopropionaldehyde derivatives of the formula Ib 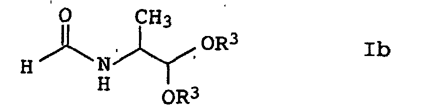 in which the substituent R³ is C₁-C₈-alkyl.

8. A process as claimed in any of claims 2 to 4, wherein the amount of the respective carboxamide R¹-CONH₂ and carboxylic acid R⁴-COOH employed is from 250 to 800 mol%, based on the acetal of the formula II employed.

9. A process as claimed in claim 8, wherein the amount of the respective carboxamide R¹-CONH₂ and carboxylic acid R⁴-COOH employed is from 400 to 600 mol%, based on the acetal of the formula II employed.

10. A process as claimed in any of claims 8 or 9, wherein the carboxamide R¹-CONH₂ and the carboxylic acid R⁴-COOH are employed in the reaction in a molar ratio of 1:1.

11. A process as claimed in any of claims 5 to 7, wherein the amount of the respective carboxamide R¹-CONH₂ and carboxylic acid R⁴-COOH employed is from 50 to 250 mol%, based on the acetal of the formula II employed.

12. A process as claimed in claim 11, wherein the amount of the respective carboxamide R¹-CONH₂ and carboxylic acid R⁴-COOH employed is from 100 to 200 mol%, based on the acetal of the formula II employed.

13. A process as claimed in either of claims 11 or 12, wherein the carboxamide R¹-CONH₂ and the carboxylic acid R⁴-COOH are employed in the reaction in a molar ratio of 1:1.

## Revendications

1. Procédé pour la préparation de dérivés N-acylés de formule générale I 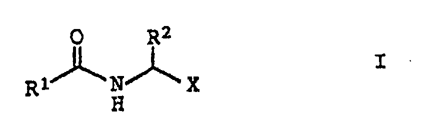 dans laquelle les symboles ont, indépendamment les uns des autres, les significations suivantes :
X : CH(OR³)₂, COOR³ ;
R¹ : l'hydrogène, un groupe alkyle en C1-C12, aryle, éventuellement substitué ;
R² : l'hydrogène, un groupe alkyle en C1-C12, aryle, éventuellement substitué ;
R³ : un groupe alkyle en C1-C12,
et dans le cas où le symbole X représente CH(OR³)₂, les substituants R³ peuvent également former un cycle à cinq ou six chaînons avec les atomes d'oxygène auxquels ils sont reliés,
**caractérisé par le fait que** l'on fait réagir un carboxamide R¹-CONH₂ de formule générale II avec un dérivé de monoacétal du glyoxal de formule générale III 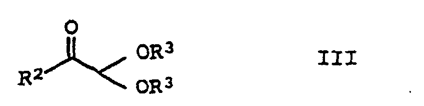 en présence d'un acide carboxylique R⁴-COOH de formule génétrale IV dans laquelle R⁴ représente l'hydrogène ou un groupe alkyle en C1-C12, les symboles R¹ à R³ ayant les significations indiquées ci-dessus.

2. Procédé selon la revendication 1, **caractérisé par le fait que** les symboles ont les significations suivantes :
R¹ : l'hydrogène, un groupe alkyle en C1-C8 ;
R² : un groupe alkyle en C1-C8, aryle, éventuellement substitué ;
R³ et R⁴
des groupes alkyle en C1-C8.

3. Procédé selon la revendication 2, **caractérisé en ce que** les symboles ont les significations suivantes
R¹ : l'hydrogène ;
R² à R⁴ :
des groupes alkyle en C1-C8.

4. Procédé selon l'une des revendications 2 ou 3, pour la préparation des esters N-formyl-alpha-amino-propioniques de formule générale Ia 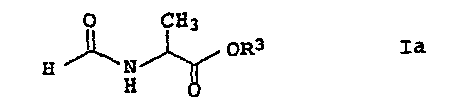 dans laquelle R³ représente un groupe alkyle en C1-C8.

5. Procédé selon la revendication 1, **caractérisé par le fait que** les symboles ont les significations suivantes
X : CH(OR³)₂ ;
R¹ : l'hydrogène, un groupe alkyle en C1-C8 ;
R² : un groupe alkyle en C1-C8, aryle, éventuellement substitué ;
R³ et R⁴ :
des groupes alkyle en C1-C8.

6. Procédé selon la revendication 5, **caractérisé par le fait que** les symboles ont les significations suivantes :
R¹ : l'hydrogène ;
R² à R⁴ :
des groupes alkyle en C1-C8.

7. Procédé selon l'une des revendications 5 ou 6 pour la préparation de dérivés du N-formyl-2-amino-propionaldéhyde de formule générale Ib 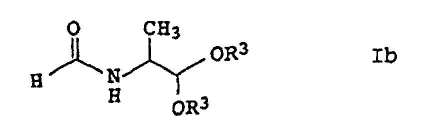 dans laquelle R³ représente un groupe alkyle en C1-C8.

8. Procédé selon l'une des revendications 2 à 4, **caractérisé par le fait que** la quantité du carboxamide R¹-CONH₂ mis en oeuvre et de l'acide carboxylique R⁴-COOH mis en oeuvre est de 250 à 800 mol %, par rapport à l'acétal de formule générale II mis en oeuvre, pour chacun d'entre eux.

9. Procédé selon la revendication 8, **caractérisé par le fait que** la quantité du carboxamide R¹-CONH₂ mis en oeuvre et de l'acide carboxylique R⁴-COOH mis en oeuvre est de 400 à 600 mol %, par rapport à l'acétal de formule générale II mis en oeuvre, pour chacun d'entre eux.

10. Procédé selon l'une des revendications 8 ou 9, **caractérisé par le fait que** le carboxamide R¹-CONH₂ et l'acide carboxylique R⁴-COOH sont mis en oeuvre dans -la réaction à un rapport molaire de 1 : 1.

11. Procédé selon l'une des revendications 5 à 7, **caractérisé par le fait que** la quantité du carboxamide R¹-CONH₂ mis en oeuvre et de l'acide carboxylique R⁴-COOH mis en oeuvre est de 50 à 250 mol %, par rapport à l'acétal de formule générale II mis en oeuvre, pour chacun d'entre eux.

12. Procédé selon revendication 11, **caractérisé par le fait que** la quantité du carboxamide R¹-CONH₂ mis en oeuvre et de l'acide carboxylique R⁴-COOH mis en oeuvre est de 100 à 200 mol %, par rapport à l'acétal de formule générale II mis en oeuvre, pour chacun d'entre eux.

13. Procédé selon l'une des revendications 11 ou 12, **caractérisé en ce que** le carboxamide R¹-CONH₂ et l'acide carboxylique R⁴-COOH sont mis en oeuvre dans la réaction un rapport molaire de 1 : 1.
